# EUROPEAN PATENT APPLICATION

(11) **EP 3 954 378 A1**
(43) Date of publication of application: **16.02.2022**
(21) Application number: 20190515.5
(22) Date of filing: 11.08.2020
(51) Int. Cl.: A61K 35/17, A61P 29/00, A61P 11/00, A61K 9/00, A61M 11/00, C12N 5/0783

(54) **COMPOSITIONS COMPRISING T-CELLS FOR TOPICAL ADMINISTRATION TO THE LUNG**

(71) Applicant: Sandoz AG, 4056 Basel (CH)
(72) Inventor: LITTRINGER, Eva, 6330 Kufstein (AT); LAMBERT, Katharina, 4643 Pettenbach (AT)
(74) Representative: Kluschanzoff, Harald

(57) **Abstract**

The present invention relates to a composition comprising T-cells for use in treatment and/or prevention of an immune imbalance in a subject by topical administration to the lung. The present invention further relates to a kit comprising the aforesaid composition and a means of administration, as well as to a device adapted for aerosolization of a composition comprising cells, wherein said device comprises the aforesaid composition comprising T-cells.

## Description

The present invention relates to a composition comprising T-cells for use in treatment and/or prevention of an immune imbalance in a subject by topical administration to the lung. The present invention further relates to a kit comprising the aforesaid composition and a means of administration, as well as to a device adapted for aerosolization of a composition comprising cells, wherein said device comprises the aforesaid composition comprising T-cells.

T cells play a crucial role in the regulation of the immune system and in balancing activation vs. moderation of immune responses. In particular, regulatory T cells (Tregs) are a major subset of CD4+ T cells that suppress the action of auto-reactive pro-inflammatory effector cells, contribute to tissue repair and are considered a promising tool to restore immune homeostasis (Rakebrandt et al (2016), Swiss Med Weekly 146:w14343). In particular CD4+FOXP3+ regulatory T cells (Tregs) play a crucial role in maintaining immune homeostasis by suppressing pro-inflammatory cells of the immune system including CD4+ T-helper cells and CD8+ cytotoxic T cells. Novel treatment approaches involve parenteral delivery of autologous, in vitro expanded polyclonal Treg cells, which have undergone extensive testing through clinical trials (Ferreira et al. (2019), Nat Rev Drug Discov 18(10):749). While this technique has been proven safe, its efficacy may be limited by suboptimal delivery of cells to the site of inflammation. In addition, chimeric antigen receptor technology and its application to Tregs harbors great therapeutic potential to allow targeted treatment of diseases with underlying immune imbalance.

To date, chronic inflammatory diseases, including COPD and autoimmune diseases such as type 1 diabetes, multiple sclerosis or rheumatoid arthritis, affect one in five people in the western population and constitute a significant burden on federal health care systems. Autoimmune processes are driven by an imbalance of pro-and anti-inflammatory cell subsets of the immune system, leading to tissue destruction and long-term disabilities affecting various organs including the respiratory system. Lack of properly balanced Treg numbers and function are major drivers in the development of chronic inflammatory diseases (e.g. COPD, Jin et al. (2014), PLoS One 9(10):e111044; Chiappori et al. (2016), World Allergy Org J 9:5, Wu et al. (2019), Frontiers Immunol 10:Art. 220) and autoimmune diseases, while their accumulation during chronic viral infections or in tumor environments may prevent viral or tumor clearance.

Also, the probability of being diagnosed with cancer is approx. 30%. Novel approaches in cancer treatment involve immunotherapy, specifically adoptive cell transfer (ACT). Among ACT approaches, chimeric antigen receptor (CAR) T cell therapy (cf. e.g. Tisagenlecleucel (Kymriah^{®}, and similar preparations) has achieved remarkable responses in certain patient groups. CAR T cells are genetically engineered to express T cell receptors that specifically recognize an antigen of interest such as tumor antigens (e.g. Fransson et al. (2012), J Neuroinflammation 9:112). While this technique has been proven safe, largely in clinical trials with leukemic patients, its efficacy to target tumors may depend on delivery of cells to the site of tumor persistence, such as the respiratory system (Stephan et al. (2015), Nat. Biotechnol. 33(1):97; Tchou et al. (2017), Cancer Immunol. Res. 5(12):1152; Adusumilli et al (2019), AACR Annual Meeting 2019, Presentation CT036).

Local administration of therapeutic compounds to the lung, e.g. by inhalation, has been known for centuries. Although traditionally used mostly with small molecule compounds, recent developments provided methods for administration of high-molecular weight compounds, or even cells (Röhm et al. (2017), Int J Pharmaceutics 532:537; Cartez-Jugo et al. (2015), Biomicrofluidics 9:052603; Beneke et al. (2018), Scientific Reports 8:16281).

There is, nonetheless, still a need for improved means and methods for providing therapy of immune imbalances, in particular in the lung, preferably avoiding the disadvantages of the prior art. The technical problem is solved by the embodiments characterized in the claims and described herein below.

In accordance, the present invention relates to a composition comprising T cells for use in treatment and/or prevention of an immune imbalance in a subject by by topical administration to the lung.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Also, as is understood by the skilled person, the expressions "comprising a" and "comprising an" preferably refer to "comprising one or more", i.e. are equivalent to "comprising at least one".

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

As used herein, the term "standard conditions", if not otherwise indicated, relates to IUPAC standard ambient temperature and pressure (SATP) conditions, i.e. preferably, a temperature of 25°C and an absolute pressure of 100 kPa; also preferably, standard conditions include a pH of 7. Moreover, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of' means including the components specified but excluding other components except for materials present as impurities, unavoidable materials present as a result of processes used to provide the components, and components added for a purpose other than achieving the technical effect of the invention. For example, a composition defined using the phrase "consisting essentially of' encompasses any known acceptable additive, excipient, diluent, carrier, and the like. Preferably, a composition consisting essentially of a set of components will comprise less than 5% by weight, more preferably less than 3% by weight, even more preferably less than 1% by weight, most preferably less than 0.1% by weight of non-specified component(s).

The term "composition", as used herein, relates to a mixture of compounds comprising at least the T cells as specified herein and at least one carrier. The T cells comprised in the composition are described herein below. The composition preferably is a liquid which may have any consistency deemed appropriate by the skilled person. Preferably, the composition is an aqueous suspension of T cells.

The composition preferably comprises a carrier. The carrier(s) preferably is/are acceptable in the sense of being compatible with the other ingredients of the composition and being not deleterious to a potential recipient thereof. The carrier(s) preferably is/are selected so as not to affect the biological activity of the composition. Preferably, the composition is sterile, more preferably a sterile solution, most preferably a sterile solution for topical administration to the lung, in particular inhalation. The carrier is selected by the skilled person such as to achieve the consistency intended and may in particular be a liquid, more preferably an aqueous liquid. Examples of such carriers are distilled water, physiological saline, Ringer's solutions, dextrose solution, phosphate-buffered saline solution, and Hank's solution. The carrier may include one or more solvents or other ingredients increasing solubility of the compounds comprised in the composition. Further examples of liquid carriers or excipients thereof are syrup, oil such as peanut oil and olive oil, water, emulsions, various types of wetting agents, sterile solutions and the like. Suitable carriers comprise those mentioned above and others well known in the art. As is understood by the skilled person, the composition, in particular the pharmaceutical composition, may comprise one or more further compounds; preferably, such additional compounds are selected so as to not affect the biological activity of the composition, in particular of the T cells and/or are acceptable in the sense of being compatible with the other ingredients of the composition and being not deleterious to a potential recipient thereof. The composition preferably comprises a pH controlling compound, in particular a buffer; and/or an osmotic stabilizer, such as a salt and/or a sugar. Preferably, the carrier is phosphate buffered saline, more preferably further comprising an osmolyte at a concentration of from 200 to 1000 mosm, more preferably of from 250 to 600 mosm; preferably, the osmolyte is a physiologically compatible sugar, preferably is sucrose, lactose, or mannitol, more preferably is sucrose. Preferably, the carrier further comprises serum, preferably human serum, more preferably autologous serum, e.g. in a concentration (v/v) of from 1% to 20%, preferably of from 2% to 10%.

Preferably, the composition according to the present specification is a pharmaceutical composition; thus, preferably, the carrier is a pharmaceutically acceptable carrier. In addition, the pharmaceutical composition or formulation may also include other carriers, adjuvants, stabilizers and/or other compounds deemed appropriate by the skilled person, e.g. for galenic purposes. As referred to herein, the T cells as specified herein below are the "active compound" of the preparation, although "further active compounds", which are referred to under this term, may be present. Preferably, the further active compound is a pharmaceutically active compound, e.g. a chemokine, preferably a cytokine, more preferably IL-10, IL-35, IL-27, IFN-g, IL-6, type I IFNs, IL-1b, IL-23, IL-4, and/or IL-5. Also preferably, the further pharmaceutically active compound is a checkpoint inhibitor. Checkpoint inhibitors are, in principle known to the skilled person and include in particular antibodies targeting at least one the surface molecules PD-1, PD-L1, CTLA-4, TIGIT, and LAG-3. Specific pharmaceutical compositions are prepared in a manner well known in the pharmaceutical art and comprise at least the T cells referred to herein below, preferably in admixture or otherwise associated with at least one pharmaceutically acceptable carrier or diluent. For making those specific pharmaceutical compositions, the active compound(s) will usually be mixed with a carrier or the diluent. The resulting formulations are to be adapted to the mode of administration, i.e. in the forms of tablets, capsules, suppositories, solutions, suspensions or the like. Dosage recommendations shall be indicated in the prescriber's or user's instructions in order to anticipate dose adjustments depending on the considered recipient.

Suitable routes of administration conventionally used for drug administration are topical, intravenous, or parenteral administration. The pharmaceutical composition is, preferably, administered locally to the lung, e.g. via bronchoscopy or inhalation, more preferably by inhalation. Moreover, the pharmaceutical composition can be administered in combination with further active compounds either in a common pharmaceutical composition or as separated pharmaceutical compositions wherein said separated pharmaceutical compositions may be provided in form of a kit of parts.

The pharmaceutical composition is, preferably, administered in conventional dosage forms prepared by combining the T cells with standard pharmaceutical carriers according to conventional procedures. These procedures may involve mixing and/or dissolving ingredients as appropriate to obtain the desired preparation. It will be appreciated that the form and character of the pharmaceutically acceptable carrier or diluent is dictated by the amount of active ingredient with which it is to be combined, the route of administration and other well-known variables. A therapeutically effective dose refers to a number of T cells to be used in a pharmaceutical composition of the present invention which provides the effect referred to in this specification. Therapeutic efficacy and toxicity of compounds and compositions can be determined by standard pharmaceutical procedures in cell cultures or experimental animals, e.g., ED₅₀ (the dose therapeutically effective in 50% of the population) and LD₅₀ (the dose lethal to 50% of the population). The dose ratio between therapeutic and toxic effects is the therapeutic index, and it can be expressed as the ratio, LD₅₀/ED₅₀. The dosage regimen will be determined by the attending physician and other clinical factors; preferably in accordance with any one of the above described methods. As is well known in the medical arts, dosages for any one patient depend upon many factors, which may include the patient's size, body surface area, age, the particular compound to be administered, sex, time and route of administration, general health, and other drugs being administered concurrently. Progress can be monitored by periodic assessment. A typical dose can be, for example, in the range of 10³ to 10⁷, preferably of from 10⁴ to 10⁶, T cells; however, doses below or above this exemplary range are envisioned, especially considering the aforementioned factors. The pharmaceutical compositions and formulations referred to herein are administered at least once in order to treat or ameliorate or prevent a disease or condition recited in this specification. However, the said pharmaceutical compositions may be administered more than one time, for example from two to 50 times, more preferably from five to 10 times. Also preferably, the pharmaceutical composition is administered once to the subject, i.e., preferably, is used as a one-time treatment. Preferably, administration is adjusted to maintain an effective number of T cells in the body, preferably the lung, of a subject for the time period intended. In the case of chronic disease, in particular autoimmune disease, asthma, and allergy, the pharmaceutical composition is preferably administered to the patient periodically, e.g. daily, weekly, or bi weekly, over an unlimited period of time; also preferably, the pharmaceutical composition is administered pro re nata, i.e. according to the patient's need.

The term "T cells" is understood by the skilled person. Preferably, the term relates to a type of lymphocyte identifiable by the presence of a T cell receptor on the cell surface. Preferably, in particular in case the immune imbalance is an overstimulation, the T cells are CD4+ or CD8+ T cells, more preferably CD4+ T cells, still more preferably are regulatory T cells (Tregs), even more preferably are CD4+ regulatory T cells, most preferably are CAR Tregs, i.e. Treg cells carrying a CAR a specified herein below. Most preferably, in particular in case the immune imbalance is an overstimulation, the T cells are CD4+ CD127- CD25high FOXP3+ T-cells, i.e., preferably T cells characterized by presence of CD4, low levels of CD127 and high levels of CD25 on the cell surface in combination with intracellular FOXP3 expression. Thus, appropriate cells are preferably obtained by FACS sorting, using CD4, CD127, and/or CD25 as surface markers. Also preferably, in particular in case the immune imbalance is an understimulation, the T cells are a mixture of CD4+ and CD8+ T cells, more preferably are CAR T-cells, i.e., preferably T cells expressing at least one CAR as specified elsewhere herein. The T cells as specified herein may also comprise further, non-T cells, in particular other lymphocytes, preferably at least one of NK (natural killer) cells, and B cells and innate-lymphoid cells (ILCs). Further, the T cells may further comprise further, non-T cell leukocytes, preferably selected from the list consisting of neutrophils, eosinophils, basophils, and monocytes/macrophages, more preferably selected from the list consisting of neutrophils, eosinophils, and basophils. The T cells may also comprise further blood cells such as thrombocytes or erythrocytes. Preferably, however, the cells comprised in said composition are at least 50% T-cells, preferably at least 75% T-cells, more preferably at least 85% T-cells, still more preferably at least 90% T-cells, most preferably at least 95% T-cells. Also preferably, the T cells do not comprise monocytes and macrophages. Thus, preferably, the T cells are essentially pure T cells. Preferably, the T cells are autologous T-cells, in particular in case of treating an overstimulation, or are heterologous T-cells, in particular in case of treating an understimulation. Preferably, the T-cells are genetically modified; methods and targets for genetic modification in T cells are, in principle, known to the skilled person. Preferably, the genetic modification is achieved by viral or non-viral genome targeting, e.g. via the CRISPR/Cas system (cf. e.g. Roth et al. (2018), Nature 559(7714):405).

The term "chimeric antigen receptor", abbreviated as "CAR", is, in principle, known to the skilled person. Preferably, the term relates to a polypeptide comprising an extracellular binding domain, a transmembrane domain, and an intracellular signaling domain, and having the activity of stimulating a T cell carrying the CAR upon binding of a cognate binding partner to the extracellular binding domain. In accordance, the extracellular binding domain comprises at least one domain binding, preferably specifically, an antigen of interest as specified herein below. Preferably, the extracellular binding domain of the CAR is derived from an antibody directed against said antigen of interest; thus, preferably, the extracellular binding domain is a single-chain antibody, a nanobody, or any type of antibody derivative which can be expressed from a single gene. The transmembrane domain of the CAR may be any transmembrane domain deemed suitable by the skilled person, preferably is a transmembrane domain derived from CD28 or CD3, in particular the CD3ζ chain. The intracellular signaling domain preferably is adapted to induce activation of a T cell upon contact of the extracellular binding domain to the antigen of interest. Preferably, the activation domain comprises at least one activation motif from the CD3ζ chain, CD28 and/ or CD137.

The term "antigen of interest", as used herein, relates to any antigen deemed suitable by the skilled person for treating and/or preventing an immune imbalance in a subject, in particular an understimulation or overstimulation. Antigens of interest are in principle known to the skilled person and are selected according to the treatment and/or prevention intended and, preferably, according to the clinical situation. Preferably, the antigen of interest is an antigen of a cell, preferably an epitope of a surface macromolecule, more preferably of a surface polypeptide. Preferably, in case of an immune understimulation, the antigen of interest is a tumor antigen, i.e. preferably, an antigen expressed by a cancer cell, preferably by a tumor cell as specified elsewhere herein. More preferably, the antigen is a tumor specific antigen, i.e. a tumor antigen essentially only expressed by cancer cells; thus, a tumor specific antigen preferably is an antigen expressed by cancer cells but only to an at least 5fold, preferably at least 10fold, reduced amount in non-cancer cells of a subject; and/or is an antigen expressed by cancer cells but only in less than 10%, more preferably less than 1%, of non-cancer cells of a subject. Thus, preferably, the antigen of interest is at least one of mesothelin, CD19, CD30, CD33, CD123, FLT3, and BCMA, more preferably is mesothelin (cf. eg. ClinicalTrials.gov Identifier: NCT02414269). Also preferably, in case of an immune overstimulation, the antigen of interest preferably is a lung antigen, i.e. an antigen present in the lung, in particular present on lung epithelium.

The term "cancer" is, in principle, understood by the skilled person and relates to a disease of an animal, including man, characterized by uncontrolled growth by a group of body cells (cancer cells). This uncontrolled growth may form a mass of cancer cells (tumor) and may be accompanied by intrusion into and destruction of surrounding tissue (invasion) and possibly spread of cancer cells to other locations in the body (metastasis). Preferably, also included by the term cancer is tumor recurrence, i.e. relapse. Thus, preferably, the cancer is a solid cancer, i.e. a cancer forming at least one detectable tumor, is a metastasis, or is a relapse. Preferably, the cancer is selected from the list consisting of aids-related lymphoma, anal cancer, appendix cancer, astrocytoma, atypical teratoid, basal cell carcinoma, bile duct cancer, bladder cancer, brain stem glioma, breast cancer, burkitt lymphoma, carcinoid tumor, cerebellar astrocytoma, cervical cancer, chordoma, colon cancer, colorectal cancer, craniopharyngioma, endometrial cancer, ependymoblastoma, ependymoma, esophageal cancer, extracranial germ cell tumor, extragonadal germ cell tumor, extrahepatic bile duct cancer, fibrosarcoma, gallbladder cancer, gastric cancer, gastrointestinal stromal tumor, gestational trophoblastic tumor, head and neck cancer, hepatocellular cancer, hodgkin lymphoma, hypopharyngeal cancer, hypothalamic and visual pathway glioma, intraocular melanoma, kaposi sarcoma, laryngeal cancer, medulloblastoma, medulloepithelioma, melanoma, merkel cell carcinoma, mesothelioma, mouth cancer, multiple endocrine neoplasia syndrome, multiple myeloma, mycosis fungoides, nasal cavity and paranasal sinus cancer, nasopharyngeal cancer, neuroblastoma, non-hodgkin lymphoma, non-small cell lung cancer, oral cancer, oropharyngeal cancer, osteosarcoma, ovarian cancer, ovarian epithelial cancer, ovarian germ cell tumor, ovarian low malignant potential tumor, pancreatic cancer, papillomatosis, paranasal sinus and nasal cavity cancer, parathyroid cancer, penile cancer, pharyngeal cancer, pheochromocytoma, pituitary tumor, pleuropulmonary blastoma, primary central nervous system lymphoma, prostate cancer, rectal cancer, renal cell cancer, retinoblastoma, rhabdomyosarcoma, salivary gland cancer, sézary syndrome, small cell lung cancer, small intestine cancer, soft tissue sarcoma, squamous cell carcinoma, squamous neck cancer, testicular cancer, throat cancer, thymic carcinoma, thymoma, thyroid cancer, urethral cancer, uterine sarcoma, vaginal cancer, vulvar cancer, waldenström macroglobulinemia, and wilms tumor. More preferably, the cancer is lung cancer, preferably non-small cell lung cancer or small cell lung cancer.

The term "treatment" refers to an amelioration of a disease or disorder referred to herein or the symptoms accompanied therewith to a significant extent. Said treating as used herein also includes an entire restoration of health with respect to the diseases or disorders referred to herein. It is to be understood that treating as used in accordance with the present invention may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc. Preferred confidence intervals are at least 90%, at least 95%, at least 97%, at least 98% or at least 99%. The p-values are, preferably, 0.1, 0.05, 0.01, 0.005, or 0.0001. Preferably, the treatment shall be effective for at least 20%, more preferably at least 50%, even more preferably at least 60%, still more preferably at least 70%, still more preferably at least 80%, most preferably at least 90% of the subjects of a given cohort or population. As will be understood by the skilled person, effectiveness of treatment is dependent on a variety of factors including, e.g. disease stage and severity. As will be further understood, the term treatment includes measures preventing and/or delaying progression of a disease or disorder referred to herein.

The term "prevention" refers to retaining health with respect to the diseases or disorders referred to herein for a certain period of time in a subject. It will be understood that said period of time is dependent on the number of T cells which has been administered and individual factors of the subject discussed elsewhere in this specification. It is to be understood that prevention may not be effective in all subjects treated with the compound according to the present invention. However, the term requires that preferably a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools discussed elsewhere in this specification.

The term "immune imbalance", as used herein, is a broad term relating to any deviation of the immune system of a subject from normal causing at least one detectable symptom. The immune imbalance may be generalized, i.e. affect the whole body, may be regional, e.g. affecting a body part or an organ, or may be local, e.g. affecting only part of an organ or tissue.

Preferably, the immune imbalance is an immune overactivation, i.e. an activation of the immune system to an extent causing symptoms in a subject. Symptoms of immune overactivation are known to the skilled person from medical textbooks and may depend on the specific type of immune overactivation. Preferably, the immune overactivation is an immune overactivation in the lung and/or is an immune overactivation having at least one pulmonary manifestation; such immune overactivations are, in principle known to the skilled person, e.g. from Cojocaru et al. (2011), Maedica (Buchar) 6(3):224. More preferably, the immune overactivation is chronic obstructive pulmonary disease (COPD), lung fibrosis, allergic coryza, asthma, interstitial lung disease, lupus erithematosus, rheumatoid arthritis, systemic sclerosis, polymyositis/dermatomyositis, Sjögren's syndrome, mixed connective tissue disease, Wegener's granulomatosis, Churg-Strauss syndrome, Goodpasture's syndrome, ankylosing spondylitis, or type I diabetes. Most preferably, the the immune overactivation is COPD, allergic coryza, asthma, interstitial lung disease, lung fibrosis, or type I diabetes.

Preferably, the immune imbalance is an immune underactivation, i.e. a lack of activation of the immune system to an extent causing symptoms in a subject. Symptoms of immune underactivation are known to the skilled person from medical textbooks and may depend on the specific type of immune underactivation. Preferably, the immune underactivation is a state of the immune system of a subject permitting inappropriate proliferation of deleterious agents which would not be able to proliferate in case the immune system were normal. As used herein, the term "deleterious agent" includes any type of biological matter, including viruses, inappropriately proliferating in a subject to an extent causing at least one symptom; thus, the term includes cancer as specified elsewhere herein. As will be understood by the skilled person, immune underactivation may be caused by lack of activation of the immune system or cell types thereof, in particular of T cells and/or may be caused by the inability of components of the immune system to reach the site of proliferation of said deleterious agents. Thus, preferably, immune underactivation is or results in cancer or infection with an infectious agent, such as a virus or a bacterium; or the immune underactivation is caused by an underlying disease such as cystic fibrosis.

The term "subject", as used herein, relates to a multicellular animal, preferably to a vertebrate, more preferably to a mammal. More preferably, the subject is a human, a cattle, a pig, a sheep, a goat, a horse, a cat, a dog, a guinea pig, a mouse, or a rat. Preferably, the subject is a laboratory animal, preferably a guinea pig, a mouse, or a rat. Also preferably, the subject is a livestock, preferably a cattle, a pig, a sheep, a goat, or a horse. Also preferably, the subject is a companion animal, preferably a cat, a dog, or a guinea pig. Most preferably, the subject is a human. Preferably, the subject is known, suspected to, or being at risk of suffering from an immune imbalance as specified herein.

The term "topical administration" is known to the skilled person. As used herein, the term relates to an administration of a composition to or on a particular place, tissue, or organ in or on the body of a subject, regardless of the location of the effect in treatment and/or prevention. Preferably, as used herein, in topical administration also the effect in treatment and/or prevention is topical, i.e. localized, to the place, tissue, or organ the composition was administered to.

In accordance with the above, the term "topical administration to the lung", preferably, relates to a local administration to the lung, preferably to at least one bronchus, broncheolus, and/or alveolus. Thus, topical administration to the lung preferably is bronchial administration, bronchiolar administration, and/or alveolar administration. Also preferably, topical administration to the lung is administration to respiratory epithelium, in particular to respiratory mucosa. Preferably, in topical administration to the lung, the effect in treatment and/or prevention occurs in the lung, i.e. preferably is treatment and/or prevention of lung disease, lung disorder, and/or lung symptoms. Topical administration to the lung can be achieved in a number of ways in principle known to the skilled person. Preferably, topical administration to the lung is performed by lavage, via a bronchoscopy device, or by inhalation, more preferably via a bronchoscopy device or by inhalation. Preferably, topical administration to the lung comprises aerosolization of the composition, more preferably as a liquid in air aerosol, preferably with droplet sizes as specified herein below.

The term "inhalation" is known to the skilled person and relates to administration of at least one compound to a subject by intake of breath. Thus, preferably, inhalation causes administration of T cells via the respiratory tract, preferably at least into the trachea, more preferably at least into the bronchi, more preferably at least into bronchioles, most preferably into alveoli. In accordance, inhalation preferably causes the T cells to contact the broncheolar epithelium, more preferably the alveolar epithelium. Also preferably, in case the immune imbalance is a local immune imbalance, inhalation preferably causes the T cells to contact the site of immune imbalance, in particular a cancer site, e.g. a tumor, or a site of infection.

Preferably, inhalation is inhalation of drops of liquid comprising the T cells, preferably generated by a nebulizer or a pump spray device. Preferably, the average droplet size is less than 20 µm, more preferably less than 15 µm. Preferably, the average droplet size is of from 3 µm to 12 µm, more preferably of from 5 µm to 8 µm. Thus, inhalation preferably comprises aerosolization of a composition comprising T cells as specified herein above, preferably as a liquid in air aerosol.

Advantageously, it was found in the work underlying the present invention that T cells can be administered via inhalation and that this administration permits local administration into the lung.

The definitions made above apply mutatis mutandis to the following. Additional definitions and explanations made further below also apply for all embodiments described in this specification mutatis mutandis.

The present invention further relates to a kit comprising a composition comprising T-cells of the present invention and a means of administration.

The term "kit", as used herein, refers to a collection of the aforementioned components. Preferably, said components are combined within an outer container, preferably with additional components. Examples for such components of the kit as well as methods for their use have been given elsewhere in this specification. The kit, preferably, contains the aforementioned components in a ready-to-use formulation. The kit preferably comprises further components, e.g. a diluent. Preferably, the kit may additionally comprise instructions, e.g., a user's manual for carrying out the administration as specified herein. Details are to be found elsewhere in this specification. Additionally, such user's manual may provide instructions about correctly using the components of the kit. A user's manual may be provided in paper or electronic form, e.g., stored on CD or CD ROM.

The term "means of administration", as used herein, relates to any means adapted for an administration of the composition as specified herein. Preferably, the means of administration comprises an aerosolization device, preferably as specified herein below. Also preferably, the kit comprises or further comprises a means for assisting in inhalation, e.g. a face mask, a spacer, a mouthpiece, a nosepiece, and/or a nozzle. The kit may also comprise a means for administering the composition via bronchoscopy; appropriate means are known in the art. Preferably, the kit further comprises a technical means aiding in inhalation and/or aerosol generation, such as a pressure pump, a pressure container like a compressed air container, or the like.

The present invention also relates to a device adapted for aerosolization of a composition comprising cells, wherein said device comprises a composition comprising T-cells.

The term "device", as used herein, relates to a system of means adapted for aerosolization of a composition comprising cells, preferably of an aqueous suspension of cells. Means for aerosolization are known in the art. Preferred are devices permitting aerosolization with low shear forces and low heat transmission and include e.g. nebulizers, in particular vibrating mesh nebulizers, and pump spraying devices. Thus, preferably, the device comprises an aerosolization unit, preferably a vibrating mesh unit or a spray nozzle. Preferably, the device further comprises a reservoir, preferably adapted to hold a composition as specified elsewhere herein, operationally connected to the aerosolization unit. Preferably, the device comprises further components, such as means for assisting in inhalation, e.g. a face mask, a spacer, a mouthpiece, a nosepiece, and/or a nozzle, and or a means adapted for delivering the composition via bronchoscopy. Also preferably, the device further comprises technical means contributing to aerosol generation, such as a pressure pump, a pressure container such as a compressed air container, or the like. How to link the above means in an operating manner will depend on the type of means included into the device. Preferably, the means are comprised by a single device, e.g. in an inhalator device or in a pump spray device.

The present invention also relates to a method of treating an immune imbalance in a subject, comprising
(a) contacting said subject with T-cells by topical administration to the lung, and
(b) thereby treating said immune imbalance.

The method of the present invention, preferably, is an in vivo method. Moreover, it may comprise steps in addition to those explicitly mentioned above. For example, further steps may relate, e.g., to providing T cells for step a), or administration of further compounds in step b). In particular in case the T cells are CAR T cells, further treatment may relate to surveying and, if necessary, treating the subject for a cytokine storm potentially elicited by the administration of said CAR T cells. Moreover, one or more of the aforesaid steps may be performed by automated equipment.

The present invention further relates to a use of a composition comprising T-cells for the manufacture of a medicament for the treatment of an immune imbalance by topical administration to the lung.

In view of the above, the following embodiments are particularly envisaged:
Embodiment 1: A composition comprising T-cells for use in treatment and/or prevention of an immune imbalance in a subject by topical administration to the lung.
Embodiment 2: The composition for use of embodiment 1, wherein said immune imbalance is an immune imbalance in the lung of said subject.
Embodiment 3: The composition for use of embodiment 1 or 2, wherein said treatment comprises aerosolization of said T-cells.
Embodiment 4: The composition for use of any one of embodiments 1 to 3, wherein said aerosolization comprises nebulization.
Embodiment 5: The composition for use of any one of embodiments 1 to 4, wherein said composition further comprises at least one chemokine and/or checkpoint inhibitor.
Embodiment 6: The composition for use of any one of embodiments 1 to 5, wherein said immune imbalance is immune overactivation, preferably chronic obstructive pulmonary disease (COPD), allergic coryza, asthma, interstitial lung disease, lung fibrosis or autoimmune diseases including systemic autoimmune diseases with pulmonary manifestations. The autoimmune disease, preferably, is rheumatoid arthritis, multiple sclerosis, systemic lupus erythematosus, systemic sclerosis, polymyositis, dermatomyositis, Sjoegren's syndrome, mixed connective tissue disorder, ankylosing spondylitis or type 1 diabetes or is immune underactivation, preferably cancer or infection with an infectious agent.
Embodiment 7: The composition for use of embodiment 6, wherein said autoimmune disease is systemic autoimmune diseases with pulmonary manifestations or type 1 diabetes, preferably rheumatoid arthritis, multiple sclerosis, systemic lupus erythematosus, systemic sclerosis, polymyositis, dermatomyositis, Sjoegren's syndrome, mixed connective tissue disorder, or ankylosing spondylitis.
Embodiment 8: The composition for use of any one of embodiments 1 to 7, wherein the cells comprised in said composition are at least 50% T-cells, preferably at least 75% T-cells, more preferably at least 85% T-cells, still more preferably at least 90% T-cells, most preferably at least 95% T-cells.
Embodiment 9: The composition for use of any one of embodiments 1 to 8, wherein said immune imbalance is treatable by administration of regulatory T-cells.
Embodiment 10: The composition for use of any one of embodiments 1 to 9, wherein said T-cells are regulatory T-cells.
Embodiment 11: The composition for use of any one of embodiments 1 to 10, wherein said T-cells are CD4+ CD25high CD127- FOXP3+ T-cells and/or regulatory T-cells carrying a chimeric antigen receptor (CAR Tregs).
Embodiment 12: The composition for use of any one of embodiments 9 to 11, wherein said immune balance is overactivation.
Embodiment 13: The composition for use of any one of embodiments 1 to 8, wherein said immune imbalance is treatable by administration of CAR T-cells and/or genome-edited T cells.
Embodiment 14: The composition for use of embodiment 13, wherein said T-cells are genetically engineered T-cells, preferably are T-cells expressing at least one chimeric antigen receptor (CAR T-cells), more preferably are CAR Tregs.
Embodiment 15: The composition for use of any one of embodiments 11 to 14, wherein said CAR binds, preferably specifically binds, a tumor antigen, preferably a tumor-specific antigen.
Embodiment 16: The composition for use of any one of embodiments 13 to 15, wherein said immune imbalance is underactivation, preferably cancer or infection with an infectious agent.
Embodiment 17: The composition for use of any one of embodiments 1 to 16, wherein said T-cells are autologous T-cells, more preferably are heterologous T-cells.
Embodiment 18: A kit comprising a composition comprising T-cells as specified in any one of embodiments 1 to 17, and a means of administration.
Embodiment 19: A device adapted for aerosolization of a composition comprising cells, wherein said device comprises a composition comprising T-cells.
Embodiment 20: The device of embodiment 19, wherein said device is a is a pump spray device or is a nebulizer, preferably a vibrating mesh nebulizer.
Embodiment 21: A method of treating an immune imbalance in a subject, comprising
   (a) contacting said subject with T-cells topical administration to the lung, and
   (b) thereby treating said immune imbalance.
Embodiment 22: Use of a composition comprising T-cells for the manufacture of a medicament for the treatment of an immune imbalance by topical administration to the lung.

All references cited in this specification are herewith incorporated by reference with respect to their entire disclosure content and the disclosure content specifically mentioned in this specification.

### Figure Legends

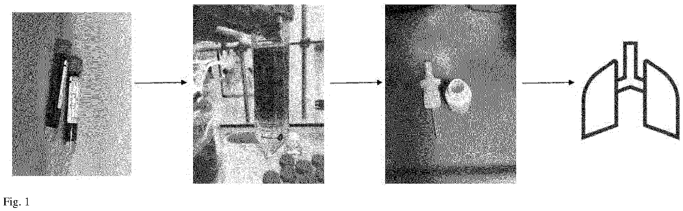

Fig. 1: Exemplary Experimental overview of separation of T-cells from blood and subsequent aerosolization
Fig. 2: Images of vital T-cells after aerosolization, Trypan blue staining; left: brightfield, right: brightfield, enhanced contrast

The following Examples shall merely illustrate the invention. They shall not be construed, whatsoever, to limit the scope of the invention.

### Example 1:

Aim: Isolation of PBMCs (peripheral blood mononuclear cells) and test if they survive aerosolization

Method: PMBCs were isolated from whole blood by Ficoll density gradient centrifugation and subsequently aerosolized through nasal spray device. Subsequently vitality is tested.

Results: vital PBMCs were obtained after aerosolization through nasal spray device

### Equipment and reagents:

Centrifuge, Hettich, Rotanta/TRC
Keyence VHX-5000 microscope equipped with a Keyence VHX-5020 camera
VWR 50 ml centrifuge tubes, VWR 89039-658
Hematocytometer, Bright-Line, Sigma-Aldrich, 2359629-1EA
Ficoll-Paque Plus, GE Healthcare, 17-14440-02; Lot. 10285499
5 ml Serological pipette, Stripette, Costar, 4487
Pipette Aids
Vials, 1.8 mL
1 ml and 200 ml pipette tips and pipette
PBS sterile, Ca2+ and Mg2+ free, Sigma, D8537-500ml, Lot. RNBJ1060
Whole blood (2x 15 ml)
Trypan blue, Sigma, T8154-100ml, Lot. RNBH9907
Actril, cold sterilant (to disinfect surfaces after work)
Nasal spray device (Aptar, provided by SDC Slovenia)

### Procedure:

1. Pool blood from 2 containers into 50 mL centrifuge tube (15 mL blood)
2. Dilute blood from step 1 with an equal amount of 1xPBS (room temperature), mix gently
3. Add 15 mL of Ficoll (room temperature) to a 50 ml conical tube
4. Gently overlay the Ficoll with 30 mL of diluted blood (from step 2) using a serological pipette
5. Centrifuge at 592 RCF (-> 1590 RPM; radius=210 mm; RZB=(RPM/1000)^2x1.118xradius (mm)) for 30 minutes at room temperature with brake off
6. Using a serological pipette aspirate and discard top half of the diluted plasma layer. Do not disturb the lymphocyte and monocyte band (PBMC layer). Collect the PBMCs from the diluted plasma/Ficoll interface using a serological pipette and transfer the cells into a new sterile 50 mL conical tube.
7. Add 1x PBS to a total volume of 45 mL and mix gently
8. Centrifuge at 330 RCF (1190 RPM) for 10 minutes at RT. Keep brake ON (brake setting 5)
9. After centrifugation, carefully aspirate and discard the supernatant without touching the pellet.
10. Resuspend cell pellet in 5 mL of 1X PBS
11. Fill cell suspension into nasal device and collect liquid after actuation from device
12. Staining cells after aerosolization from nasal spray with Trypan blue: mix cell suspension with Trypan blue (1:1), mix well with pipette.
13. Imaging cells with microscope

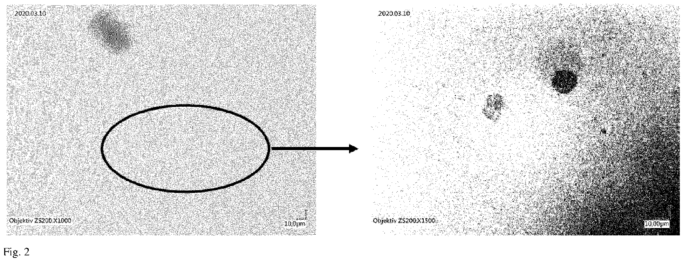

**Results:** vital PBMCs were obtained after aerosolization through the nasal spray (light grey cells in circle (Fig. 2, left), average size approx. 10 µm, Fig. 2, right)

### Literature:

- Adusumilli et al (2019), AACR Annual Meeting 2019, Presentation CT036
- Beneke et al. (2018), Scientific Reports 8:16281
- Cartez-Jugo et al. (2015), Biomicrofluidics 9:052603
- Chiappori et al. (2016), World Allergy Org J 9:5
- ClinicalTrials.gov Identifier: NCT02414269
- Cojocaru et al. (2011), Maedica (Buchar) 6(3):224
- Ferreira et al. (2019), Nat Rev Drug Discov 18(10):749
- Fransson et al. (2012), J Neuroinflammation 9:112
- Jin et al. (2014), PLoS One 9(10):e111044
- Rakebrandt et al (2016), Swiss Med Weekly 146:w14343
- Röhm et al. (2017), Int J Pharmaceutics 532:537
- Roth et al. (2018), Nature 559(7714):405
- Stephan et al. (2015), Nat. Biotechnol. 33(1):97
- Tchou et al. (2017), Cancer Immunol. Res. 5(12):1152;
- Wu et al. (2019, Frontiers Immunol 10:Art. 220

## Claims

1. A composition comprising T-cells for use in treatment and/or prevention of an immune imbalance in a subject by topical administration to the lung.

2. The composition for use of claim 1, wherein said treatment and/or prevention comprises aerosolization of said T-cells.

3. The composition for use of claim 1 or 2, wherein said immune imbalance is an immune imbalance in the lung of said subject.

4. The composition for use of any one of claims 1 to 3, wherein said immune imbalance is immune overactivation, preferably chronic obstructive pulmonary disease (COPD), allergic coryza, asthma, interstitial lung disease, lung fibrosis, rheumatoid arthritis, multiple sclerosis, systemic lupus erythematosus, systemic sclerosis, polymyositis, dermatomyositis, Sjoegren's syndrome, mixed connective tissue disorder, ankylosing spondylitis or type 1 diabetes; or is immune underactivation, preferably cancer or infection with an infectious agent.

5. The composition for use of any one of claims 1 to 4, wherein the cells comprised in said composition are at least 50% T-cells, preferably at least 75% T-cells, more preferably at least 85% T-cells, still more preferably at least 90% T-cells, most preferably at least 95% T-cells.

6. The composition for use of any one of claims 1 to 5, wherein said T-cells are regulatory T-cells, preferably CD4+CD127-CD25high FOXP3+ T-cells, and/or are regulatory T-cells expressing a chimeric antigen receptor.

7. The composition for use of claim 6, wherein said immune imbalance is overactivation.

8. The composition for use of any one of claims 1 to 5, wherein said T-cells are genetically engineered T-cells, preferably are T-cells expressing at least one chimeric antigen receptor (CAR T-cells), more preferably are CAR Tregs.

9. The composition for use of claim 8, wherein said CAR binds, preferably specifically binds, a tumor antigen, preferably a tumor-specific antigen.

10. The composition for use of claim 8 or 9, wherein said immune imbalance is underactivation, preferably cancer or infection with an infectious agent.

11. The composition for use of any one of claims 1 to 10, wherein said T-cells are autologous T-cells, more preferably are heterologous T-cells.

12. A kit comprising a composition comprising T-cells as specified in any one of claims 1 to 11, and a means of administration.

13. The kit of claim 12, wherein said means of administration is a pump spray device or is a nebulizer, preferably a vibrating mesh nebulizer.

14. A device adapted for aerosolization of a composition comprising cells, wherein said device comprises a composition comprising T-cells.

15. The device of claim 14, wherein said device is a is a pump spray device or is a nebulizer, preferably a vibrating mesh nebulizer.
